Europäisches Patentamt

European Patent Office

Office européen des brevets

(11) Veröffentlichungsnummer: **0 025 116**
A1

(12) # EUROPÄISCHE PATENTANMELDUNG

(21) Anmeldenummer: 80104578.2

(22) Anmeldetag: 02.08.80

(51) Int. Cl.³: **G 01 N 33/15**

(30) Priorität: 04.09.79 DE 2935634

(43) Veröffentlichungstag der Anmeldung:
18.03.81 Patentblatt 81/11

(84) Benannte Vertragsstaaten:
AT BE CH DE FR GB IT LI NL SE

(71) Anmelder: Battelle-Institut e.V.
Am Römerhof 35
D-6000 Frankfurt Main 90(DE)

(72) Erfinder: Lorenz, Peter, Dr. med.
Georg-Speyer-Strasse 79
D-6000 Frankfurt Main 90(DE)

(72) Erfinder: Schwaier, Anita, Dr.
Oberortstrasse 24
D-6236 Eschborn(DE)

(74) Vertreter: Blum, Klaus-Dieter, Dipl.-Ing.
Am Römerhof 35
D-6000 Frankfurt/Main 90(DE)

(54) **Verfahren zur Prüfung auf Inaktivierung von Impfstoffen und Unschädlichkeit von Blutprodukten sowie der Wirksamkeit von Chemotherapeutika bzw. Desinfektionsmitteln gegen Virushepatit.**

(57) Zur Prüfung der Inaktivierung von Impfstoffen und Unschädlichkeit von Blutprodukten sowie der Wirksamkeit von Chemotherapeutika bzw. Desinfektionsmitteln gegen Virushepatiten werden Spitzhörnchen (Tupaia belangeri) als Tiermodell verwendet. Die Antikörperbestimmungen werden bei Virushepatitis Type A für die Dauer von 150 Tagen, bei Virushepatitis Typ B für die Dauer von 60 Tagen in bestimmten Intervallen durchgeführt.

EP 0 025 116 A1

389-99/32/79/CASCH/KRI                    21. Juli 1980


BATTELLE - INSTITUT E.V., Frankfurt/Main


==================================================================
Verfahren zur Prüfung auf Inaktivierung von Impfstoffen
und Unschädlichkeit von Blutprodukten sowie der Wirksamkeit von Chemotherapeutika bzw. Desinfektionsmitteln
                    gegen Virushepatiten
==================================================================


Die Erfindung betrifft ein Verfahren zur Prüfung auf Inaktivierung von Impfstoffen und Unschädlichkeit von Blutprodukten sowie der Wirksamkeit von Chemotherapeutika bzw.
Desinfektionsmitteln gegen Virushepatiten, insbesondere
Typ A und Typ B, durch Bestimmung von Antikörpern am Tiermodell. Sie bezieht sich ferner auf ein Verfahren zur Vermehrung und Gewinnung von Virushepatitis-Antigen, insbesondere Typ A und Typ B am Tiermodell.

Bei der Inaktivierung von Impfstoffen wird dem Impfstoff
die Vermehrungsfähigkeit genommen, wobei den Erregern die
antigene Potenz und damit die Fähigkeit, den Impfling zu
immunisieren, erhalten bleibt. Die Prüfung auf Inaktivierung ist sowohl im Hinblick auf die Wirksamkeit als auch
auf die Unschädlichkeit bzw. die Toxizität des Impfstoffs
von großer Bedeutung. Ferner werden aus Spenderblut gewonnene Produkte, wie Gerinnungsfaktoren, Albumine und Globuline, Patienten für die Heilung unterschiedlichster Krank-

- 2 -

heiten oder auch zur Kreislauftherapie verabreicht. Um sicherzugehen, daß diese Materialien keine Hepatitisviren enthalten, müssen sie vorher an einem geeigneten Tiermodell untersucht werden.

Von gleicher Bedeutung wie die Unschädlichkeit von Impfstoffen und Blutprodukten ist die Wirksamkeit von Chemotherapeutika, die für Anzahl und Größe der zu verabreichenden Dosen entscheidend ist und damit wiederum die Verträglichkeit des Präparats beim Patienten beeinflußt.

Für Arbeiten in Laboratorien, Krankenhäusern und dergleichen müssen Desinfektionsmittel bereitstehen, um eventuell vorhandenes infektiöses Material damit abtöten zu können. Z.Z. werden formalinhaltige Desinfektionslösungen gegen Virushepatitis eingesetzt, die jedoch wegen starker Geruchsbelästigung oder bei längerer Einwirkung wegen der Möglichkeit der Krebsentstehung in der Anwendung problematisch sind. Zur Prüfung der Wirksamkeit neuerer Entwicklungen auf diesem Gebiet sind ebenfalls Tiermodelle notwendig.

Zu den bisher bekannten Tiermodellen für die Virushepatitis Typ A gehören Krallenaffen und Menschenaffen der Arten S. mystax, S. nigricollis, S. fuscicollis, S. oedipus, Callithrix jacchus, C. argentata, Cercopithecus aethiops, Pantroglodytes und Anthropopithecus troglodytes. Für die Virushepatitis Typ B sind Primaten, z.B. Schimpansen, bisher das einzige bekannte Tiermodell. Alle diese Tiermodelle sind zu kostspielig im Unterhalt und teilweise vom Aussterben bedroht. Ferner ist die Züchtung zu unwirtschaftlich, da z.B. bei C. jacchus pro Zuchtpaar im Jahr günstigenfalls vier Nachwuchstiere gezüchtet werden können. Andere besser verfügbare Subprimaten oder Primaten als Tiermodelle für Hepatitisviren sind nicht bekannt.

Der Erfindung liegt daher die Aufgabe zugrunde, ein verläßliches und wirtschaftliches Verfahren zur Prüfung von Impfstoffen, Blutprodukten, Chemotherapeutika und Desinfektionsmitteln zu schaffen, das die oben genannten Nachteile nicht aufweist. Es sollte ein Tiermodell gefunden werden, das zur Vermehrung von Hepatitisviren geeignet ist.

Es hat sich nun gezeigt, daß sich diese Aufgabe lösen läßt, wenn die Antikörperbestimmungen an Spitzhörnchen (Tupaia belangeri) vorgenommen werden. Zur Vermehrung und Gewinnung von Virushepatitis-Antigen werden Spitzhörnchen mit Hepatitisviren infiziert und das Antigen wird aus Organen und Ausscheidungen der infizierten Tiere oder aus Zellkulturen, die aus Organen der Tiere angelegt werden, in an sich bekannter Weise gewonnen. Somit läßt sich ein Ausgangsprodukt für Impfstoffe gegen Hepatitiserreger herstellen.

Bei der Prüfung von Impfstoffen, Blutprodukten, Chemotherapeutika und Desinfektionsmitteln werden die Spitzhörnchen auf Bildung der spezifischen Antikörper untersucht. Bei Virushepatitis Typ A werden die Antikörperbestimmungen für die Dauer von 150 Tagen, vorzugsweise wöchentlich, vorgenommen. Bei Virushepatitis Typ B werden sie für die Dauer von 60 Tagen in bestimmten Abständen, und zwar beginnend spätestens am vierten Tag nach der Infektion, zweimal während der ersten Woche, dann wöchentlich durchgeführt. Dabei handelt es sich um die Untersuchung auf Bildung von Antikörpern gegen das Oberflächenantigen (HBsAg; Anti-HBs) und gegen das innere Antigen (HBcAg; Anti-HBc). Durch eine Immunsuppression, z.B. durch intramuskuläre Impfung von Hostacortin (5 mg/kg) und Endoxan (10 mg/kg) an alternierenden Tagen, angefangen drei Tage vor der Infektion und durchgeführt bis zum siebten Tag nach der Infektion, wird das Angehen der Infektion gefördert, so daß bereits zwei Wochen nach der Infektion Antikörper gegen HBcAg nachweisbar sind.

Spitzhörnchen sind stammesgeschichtlich hochentwickelt und gehören zu den Subprimaten. Im Vergleich zu den Affen, insbesondere im Hinblick auf die Zucht, sind sie vorteilhafter. Aus einem Spitzhörnchen-Zuchtpaar können im Jahr bis zu 20 Nachwuchstiere hervorgehen. Es hat sich gezeigt, daß die Tiere die Eigenschaft haben, auf die intravenöse Verabreichung von Fremdproteinen oder Antigenen mit einer bereits vier Tage später nachweisbaren spezifischen Antikörperbildung zu reagieren. Diese sofortige Immunantwort zeigt sich bei Infektion mit Hepatitisvirus Typ B und ist charakteristisch für die Bildung von Antikörpern gegen das Oberflächenantigen der Viren (HBsAg). Die Antikörperbildung gegen das innere Antigen (HBcAg) setzt dagegen mit einer Verzögerung von mindestens 16 Tagen ein. Die Reaktion auf das HBsAg bedeutet eine schnelle Immunantwort auf ein Fremdprotein im Sinne einer aktiven Immunisierung mit einem Tot-Impfstoff. Die zeitlich verzögerte Reaktion auf das HBcAg dagegen deutet an, daß sich das Antigen im Versuchstier vermehrt hat, ehe es eine Antikörperbildung hervorrufen konnte. Der Nachweis des Anti-HBcAg-Antikörpers ist daher ein Beweis für die Infektiosität des zu untersuchenden Impfstoffs bzw. des Blutprodukts. Werden die Tiere mit Virushepatitis Typ A infiziert, so setzt die Immunantwort ebenfalls später ein und zeigt damit eine Vermehrung des Erregers in dieser Tierart an.

Bei der Prüfung von Substanzen auf antivirale Wirkung gegen Hepatitisviren werden den infizierten Tieren Chemotherapeutika verabreicht und nach bekannten Techniken Antikörperbestimmungen durchgeführt.

Für die Sicherheitsprüfung von Desinfektionsmitteln setzt man eine bestimmte Menge infektiöser Viruspartikel in verschiedenen Konzentrationen unter verschiedenen Bedingungen, wie verschiedene Temperaturen und Zeiten, solcher Mittel zu, zentrifugiert anschließend die Viruspartikel aus der Suspension aus, resuspendiert sie in einem physiologischen Puffer und verimpft sie auf das Tiermodell. Erkrankt das Tier an der Virushepatitis, so sind die Erreger durch die Behandlung mit der Desinfektionslösung nicht abgetötet worden. Daraus ergibt sich, daß die Desinfektionslösung nicht für die Inaktivierung der Hepatitisviren geeignet ist.

Die Erfindung wird durch die folgenden Beispiele näher erläutert:

Beispiel 1

Vier Spitzhörnchen werden je 0,5 ml einer Suspension von Hepatitisviren Typ B, die durch Saccharosegradientenzentrifugation gereinigt, in Schimpansenplasma und anschließend mit 1,5 Volumen einer Salzlösung nach Hanks verdünnt worden war, intravenös eingeimpft. Zwei Tiere werden immunsuppressiv nach der oben beschriebenen Methode behandelt. Angefangen am 4. Tag nach der Infektion und vom 7. Tag nach der Infektion an in wöchentlichen Abständen werden Serumproben gewonnen und auf den Gehalt an Antikörpern gegen HBcAg und HBsAg untersucht. Bei allen vier Tieren sind bereits am 4. Tag nach der Infektion Antikörper gegen HBsAg nachweisbar. Bei den Tieren, die nicht immunsuppressiv behandelt worden sind, werden dagegen erst am 20. bzw. 42. Tag nach der Infektion Antikörper gegen HBcAg nachgewiesen. Nach der Infektion unter Immunsuppression sind Antikörper gegen HBcAg bereits 14 Tage nach der Infektion nachweisbar. Die pathohistologische Untersuchung nach Beendigung des Versuchs zeigt, daß die Lebern der Versuchstiere in typischer Weise geschädigt waren.

Beispiel 2

Zwei Spitzhörnchen werden intravenös mit Hepatitisvirus Typ A infiziert. Anschließend werden vom 4. Tag der Infektion an wöchentliche Serumproben auf die Bildung spezifischer Antikörper untersucht. Im Gegensatz zu der schnell einsetzenden Immunantwort nach der Impfung von Hepatitisvirus Typ B können erst 35 bzw. 42 Tage nach der Infektion geringe Mengen von Antikörpern gegen Hepatitisvirus Typ A nachgewiesen werden. Die Ergebnisse zeigen, daß bis zum 150. Tag nach der Infektion die Antikörperbildung zunimmt. Am 91. Tag nach der Infektion findet man bei den Tieren leicht erhöhte Serumtransaminasenwerte (SGOT bzw. SGOT und SGPT), die eine infektionsbedingte Schädigung der Leber andeuten. Aus der spät einsetzenden Immunantwort und den erhöhten Serumtransaminasenwerten geht hervor, daß das Spitzhörnchen ein für die Prüfung der Wirksamkeit von Substanzen zur Bekämpfung der Virushepatitis Typ A geeignetes Veruchstiermodell ist. Die nach Beendigung des Versuchs durchgeführte pathohistologische Untersuchung zeigt, daß - wie in Beispiel 1 angegeben - die Lebern der Versuchstiere in typischer Weise geschädigt waren.

389-99/CASCH/KRI                              21. Juli 1980


BATTELLE - INSTITUT E.V., Frankfurt/Main


Patentansprüche

1. Verfahren zur Prüfung der Inaktivierung von Impfstoffen und Unschädlichkeit von Blutprodukten sowie der Wirksamkeit von Chemotherapeutika bzw. Desinfektionsmitteln gegen Virushepatiten, insbesondere Typ A und Typ B, durch Bestimmung von Antikörpern am Tiermodell, dadurch gekennzeichnet, daß die Antikörperbestimmungen an Spitzhörnchen (Tupaia belangeri) vorgenommen werden.

2. Verfahren zur Vermehrung und Gewinnung von Virushepatitis-Antigen, insbesondere Virushepatitis-Antigen Typ A und Typ B am Tiermodell, dadurch gekennzeichnet, daß Spitzhörnchen (Tupaia belangeri) mit Hepatitisviren infiziert und das Antigen aus Organen und Ausscheidungen der infizierten Tiere oder aus Zellkulturen, die daraus angelegt werden, gewonnen wird.

3. Verfahren nach Anspruch 1 oder 2, dadurch gekennzeichnet, daß die Antikörperbestimmungen bei Virushepatitis Typ A für die Dauer von 150 Tagen, bei Virushepatitis Typ B für die Dauer von 60 Tagen in bestimmten Abständen vorgenommen werden.

4. Verfahren nach Anspruch 3, dadurch gekennzeichnet, daß die Antikörperbestimmungen bei Virushepatitis Typ B zweimal während der ersten Woche nach der Infektion, beginnend spätestens am vierten Tag nach der Infektion, durchgeführt werden.

**0025116**

Nummer der Anmeldung

EP 80 10 4576

**EUROPÄISCHER RECHERCHENBERICHT**

Europäisches
Patentamt

| | EINSCHLÄGIGE DOKUMENTE | | KLASSIFIKATION DER ANMELDUNG (Int Cl ) |
|---|---|---|---|
| Kategorie | Kennzeichnung des Dokuments mit Angabe, soweit erforderlich, der maßgeblichen Teile | betrifft Anspruch | |
| | US - A - 3 105 011 (W. McLEAN et al.)<br><br>* Spalte 13, Zeilen 33-39 *<br><br>-- | 1 | G 01 N 33/15 |
| | DE - A - 2 621 276 (MERCK & CO)<br><br>* Seite 11, Beispiel 5 *<br><br>-- | 1,3 | |
| | CHEMICAL ABSTRACTS, Band 82, Nr. 3, 20. Januar 1975, Seite 348, Zusammenfassung Nr. 14910b, Columbus, Ohio, US,<br>J.R. BOOTH et al.: "Separation of hepatitis-associated antigen (HAA) from human plasma for the production of rabbit-anti HAA"<br>& VOX SANG 1974, 27(3), 227-31<br><br>* Zusammenfassung *<br><br>-- | 1 | RECHERCHIERTE SACHGEBIETE (Int Cl.)<br><br>G 01 N 33/15<br>A 61 K 39/29 |
| | CHEMICAL ABSTRACTS, Band 75, Nr. 1, 5. Juli 1971, Seite 268, Zusammenfassung Nr. 3202u, Columbus, Ohio, US,<br>P.C. DAS et al.: "Method for production of antibody to hepatitis-associated antigen in rabbits"<br>& BRIT.J. HAEMATOL., 1971, 363-7<br><br>* Zusammenfassung * | 1 | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 04-12-1980 | WALLINDER |

EPA form 1503.1 06.78